# EUROPEAN PATENT SPECIFICATION

(11) **EP 3 656 301 B1**
(45) Date of publication and mention of the grant of the patent: **07.07.2021**
(21) Application number: 19210964.3
(22) Date of filing: 22.11.2019
(51) Int. Cl.: A61B 5/367, A61B 5/06, A61B 5/00

(54) **COMPENSATING FOR ARTIFACTS WHILE TRACKING AN INTRABODY PROBE**
KOMPENSATION VON ARTEFAKTEN WÄHREND DER VERFOLGUNG EINER INTRAKÖRPERSONDE
COMPENSATION D'ARTÉFACTS PENDANT LE SUIVI D'UNE SONDE INTRACORPORELLE

(30) Priority: 23.11.2018 US 201816198933
(43) Date of publication of application: 27.05.2020
(73) Proprietor: Biosense Webster (Israel) Ltd., Yokneam, 2066717 (IL)
(72) Inventor: ALTMANN, Andres Claudio, 2066717 Yokneam (IL); GOVARI, Assaf, 2066717 Yokneam (IL); GLINER, Vadim, 2066717 Yokneam (IL)
(74) Representative: Small, Gary James

(56) References cited:
- US-A- 6 050 267
- US-A1- 2004 254 437
- US-A1- 2013 226 169

## Description

### FIELD OF THE INVENTION

The present invention is related to invasive medical procedures, such as electroanatomical mapping or ablation procedures.

### BACKGROUND

In some procedures, an intrabody probe is inserted into the body of a subject, and the probe is subsequently used to gather physiological information from the subject and/or to operate on the subject. In such procedures, it is helpful to track the position of the probe.

One type of tracking system includes three pairs of electrode patches, which are coupled to the subject such that the pairs are oriented along different respective axes that are orthogonal to each other. The system further includes a sensing electrode, which is coupled to the probe, and an additional electrode patch (or "ground electrode"), which is coupled to the subject's body and is connected to a suitable ground terminal. As the probe moves within the subject, respective alternating voltages are applied between the pairs of patches, such that alternating electric currents flow through the body of the subject. The sensing electrode thus attains, for each of the applied voltages, a sensing-electrode voltage (SEV) that is a function of the impedance of the subject's tissue between the sensing electrode and each of the activated electrodes. (In other words, the system implements a voltage divider in which the SEV is the output voltage.) The SEVs with respect to the ground terminal are measured, and the location of the sensing electrode is derived from the measured voltages.

For example, US Patents 5,697,377 and 5,983,126 describe a system and method for catheter location mapping and related procedures. Three substantially orthogonal alternating signals are applied through the patient, directed substantially toward the area of interest to be mapped, such as the patient's heart. A catheter is equipped with a measuring electrode, which for cardiac procedures is positioned at various locations either against the patient's heart wall, or within a coronary vein or artery. A voltage is sensed between the catheter tip and a reference electrode, such as a surface electrode on the patient, which voltage signal has components corresponding to the three orthogonal applied current signals. Three processing channels are used to separate out the three components as x, y and z signals, from which calculations are made for determination of the three-dimensional location of the catheter tip within the body.

Similarly, US Patent Application Publication 2004/0254437 describes a medical system for finding and displaying the location of electrodes within the body. The electrodes may be used to measure the voltage on the heart wall and display this as an activation map on a geometry representing the heart chamber.

In US6050267A there is described a system for detecting the position of a catheter in a patient which includes three sets of excitation electrodes, with one set disposed in each of three intersecting axes.

### SUMMARY OF THE INVENTION

There is provided, in accordance with some embodiments of the present invention, a system for ascertaining a location of a sensing electrode within a body of a subject. The system includes circuitry, configured to, while the sensing electrode is connected to a sampling terminal of an analog-to-digital (A/D) converter, connect three pairs of electrodes, which are coupled to the body of the subject such that the pairs lie along different respective axes that are orthogonal to each other, in multiple different configurations, in each of which (i) at least one of the pairs is connected to respective terminals of a signal generator, such that the signal generator applies an alternating voltage between the pair, thus causing a sensing-electrode voltage to be attained at the sensing electrode, and (ii) at least one other one of the pairs is connected to a ground terminal of the A/D converter. The system further includes a processor, configured to, for each configuration, receive, from the A/D converter, a digital representation of a sampling-terminal voltage at the sampling terminal with respect to the ground terminal. The processor is further configured to identify respective amplitudes of the sensing-electrode voltage for the configurations, based on the digital representation of the sampling-terminal voltage received for each configuration, and, based on the respective amplitudes of the sensing-electrode voltage, ascertain the location.

In some embodiments, the configurations include three configurations, in each of which a different respective one of the pairs is connected to the signal generator.

In some embodiments, in each of the configurations, both of the pairs that are not connected to the signal generator are connected to the ground terminal.

In some embodiments,
the digital representation is a first digital representation and the amplitudes are sensing-electrode-voltage amplitudes,
in each of the configurations, (i) a first one of the pairs is connected to the signal generator, (ii) a second one of the pairs is connected to the ground terminal, and (iii) a third one of the pairs is connected to a reference terminal of the A/D converter,
the processor is further configured to:
for each configuration, receive, from the A/D converter, a second digital representation of a reference-terminal voltage at the reference terminal with respect to the ground terminal, and
identify respective reference-voltage amplitudes of the reference-terminal voltage for the configurations, based on the second digital representation of the reference-terminal voltage received for each configuration, and
the processor is configured to ascertain the location based on the sensing-electrode-voltage amplitudes and the reference-voltage amplitudes.

In some embodiments, the configurations include six configurations.

In some embodiments, the circuitry includes:
three switching units, configured to connect to the pairs of electrodes, respectively; and
a controller, configured to connect the pairs of electrodes in the multiple different configurations by controlling the switching units.

In some embodiments, the processor is further configured to cause the circuitry to connect the pairs of electrodes in the multiple different configurations.

There is further provided, in accordance with some embodiments of the present invention, a method for ascertaining a location of a sensing electrode within a body of a subject. The method includes, while the sensing electrode is connected to a sampling terminal of an analog-to-digital (A/D) converter, connecting three pairs of electrodes, which are coupled to the body of the subject such that the pairs lie along different respective axes that are orthogonal to each other, in multiple different configurations, in each of which (i) at least one of the pairs is connected to respective terminals of a signal generator, such that the signal generator applies an alternating voltage between the pair, thus causing a sensing-electrode voltage to be attained at the sensing electrode, and (ii) at least one other one of the pairs is connected to a ground terminal of the A/D converter. The method further includes, for each configuration, receiving, from the A/D converter, a digital representation of a sampling-terminal voltage at the sampling terminal with respect to the ground terminal. The method further includes identifying respective amplitudes of the sensing-electrode voltage for the configurations, based on the digital representation of the sampling-terminal voltage received for each configuration, and, based on the respective amplitudes of the sensing-electrode voltage, ascertaining the location.

There is further provided, in accordance with some embodiments of the present invention, a computer software product including a tangible non-transitory computer-readable medium in which program instructions are stored. The instructions, when read by a processor, cause the processor to, while a sensing electrode within a body of a subject is connected to a sampling terminal of an analog-to-digital (A/D) converter, cause circuitry to connect three pairs of electrodes, which are coupled to the body of the subject such that the pairs lie along different respective axes that are orthogonal to each other, in multiple different configurations, in each of which (i) at least one of the pairs is connected to respective terminals of a signal generator, such that the signal generator applies an alternating voltage between the pair, thus causing a sensing-electrode voltage to be attained at the sensing electrode, and (ii) at least one other one of the pairs is connected to a ground terminal of the A/D converter. The instructions further cause the processor to receive for each configuration, from the A/D converter, a digital representation of a sampling-terminal voltage at the sampling terminal with respect to the ground terminal. The instructions further cause the processor to identify respective amplitudes of the sensing-electrode voltage for the configurations, based on the digital representation of the sampling-terminal voltage received for each configuration, and, based on the respective amplitudes of the sensing-electrode voltage, ascertain a location of the sensing electrode.

The present invention will be more fully understood from the following detailed description of embodiments thereof, taken together with the drawings, in which:

### BRIEF DESCRIPTION OF THE DRAWINGS

Fig. 1 is a schematic illustration of a system for ascertaining the location of a sensing electrode within the body of a subject, in accordance with some embodiments of the present invention; and
Fig. 2 is a schematic illustration of circuitry, in accordance with some embodiments of the present invention.

### DETAILED DESCRIPTION OF EMBODIMENTS

### OVERVIEW

When using a tracking system of the type described in the Background, the accuracy of the tracking may be compromised by movement of the subject (e.g., due to respiration), drifts in the applied voltages, the introduction of other tools into the subject's body, perspiration of the subject, and/or other artifact-inducing factors. For example, if the sensing electrode is positioned within the heart, the voltages attained at the sensing electrode may vary with the respiration of the subject, due to movement of the subject's chest.

One technique for compensating for the aforementioned artifacts is to couple a reference electrode to the subject's body, or to place the reference electrode within the subject's body, such as within the subject's coronary sinus. The voltage between the reference electrode and the ground electrode may then be measured, and this reference voltage may be used to compensate for any artifacts, provided that the artifacts cause the reference voltage to vary similarly to the SEVs.

A variation of the above technique, which does not require an additional reference electrode, is to activate only one or two pairs of electrode patches at a time, and to use the non-activated pair(s) as reference electrodes. For example, while one pair of electrodes is activated, the remaining four electrodes may be shorted to each other, and the reference voltage measured at these four electrodes may be subtracted from the SEV. (Alternatively, the reference voltage may be obtained without shorting the four electrodes to each other, by averaging the respective voltages at the four electrodes.) This technique effectively creates a virtual reference electrode within the body of the subject.

Embodiments of the present invention provide yet another technique, which, advantageously, obviates the need for the ground electrode. In particular, while one or two pairs of electrodes are activated, the SEV is measured with respect to the remaining one or two non-activated pairs. This technique effectively creates a virtual ground within the subject's body, which is affected by artifacts similarly to the SEV.

For example, while the x-axis pair is activated, the y-axis pair and z-axis pair may be connected to the ground terminal. Subsequently, the y-axis pair may be activated, while the x-axis pair and z-axis pair are connected to the ground terminal. Subsequently, the z-axis pair may be activated, while the x-axis pair and y-axis pair are connected to the ground terminal. The location of the sensing electrode may then be derived from the resulting three SEVs.

Alternatively, while each one of the pairs is activated, one of the non-activated pairs may be connected to the ground terminal, while the other non-activated pair may be used to create a virtual reference electrode, as described above. This technique may help compensate for artifacts caused by longer-term changes, such as ongoing perspiration of the subject.

For example, while the x-axis pair is activated, the y-axis pair may be connected to the ground terminal, while the z-axis pair is used for reference. The resulting reference voltage may be used to adjust the x-axis SEV (i.e., the SEV obtained during activation of the x-axis pair), yielding a first adjusted x-axis SEV. This process may then be repeated using the z-axis pair for ground and the y-axis pair for reference, thus yielding a second adjusted x-axis SEV. The two adjusted x-axis SEVs may then be averaged with one another, yielding an averaged adjusted x-axis SEV. After repeating this process for y-axis activation and z-axis activation, the location of the sensing electrode may be derived from the three averaged adjusted SEVs.

### SYSTEM DESCRIPTION

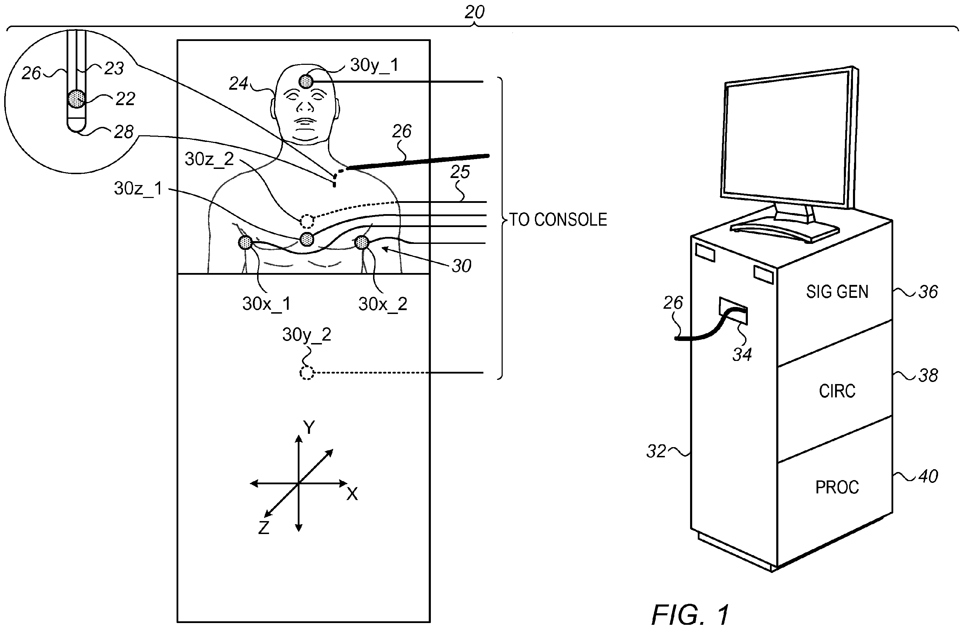

Reference is initially made to Fig. 1, which is a schematic illustration of a system 20 for ascertaining the location of a sensing electrode 22 within the body of a subject 24, in accordance with some embodiments of the present invention.

Fig. 1 depicts subject 24 undergoing a procedure in which an intrabody probe 26 is inserted into the subject's body. Such a procedure may include, for example, an ablation procedure, whereby an ablation electrode 28 at the distal end of probe 26 is used to deliver ablating currents through internal tissue (e.g., cardiac tissue) of the subject. Alternatively or additionally, such a procedure may include an electroanatomical mapping, whereby another electrode at the distal end of the probe is used to sense electrophysiological signals (e.g., cardiac electrogram signals) from the subject.

During the procedure, system 20 facilitates tracking probe 26, using at least one sensing electrode 22 that is coupled to the distal end of the probe, and three pairs of external electrodes 30. Electrodes 30 are coupled to the body of the subject such that the pairs lie along different respective axes that are orthogonal to each other. Typically, as shown in Fig. 1, these axes lie along the coronal, sagittal, and transverse planes, respectively. For example, an x-axis pair of electrodes (30x_1, 30x_2) may be coupled to respective sides of the subject (e.g., near the subject's armpits), a y-axis pair of electrodes (30y_1, 30y_2) may be coupled to the subject's head and abdomen, respectively, and a z-axis pair of electrodes (30z_1, 30z_2) may be coupled to the subject's chest and back, respectively.

Probe 26 is connected to a console 32 via an electromechanical interface 34. A wire 23, which runs through probe 26, carries signals from sensing electrode 22 to console 32. Likewise, respective wires 25 connect electrodes 30 to the console. (For simplicity, the connections of wires 25 to the console are not explicitly shown in Fig. 1.)

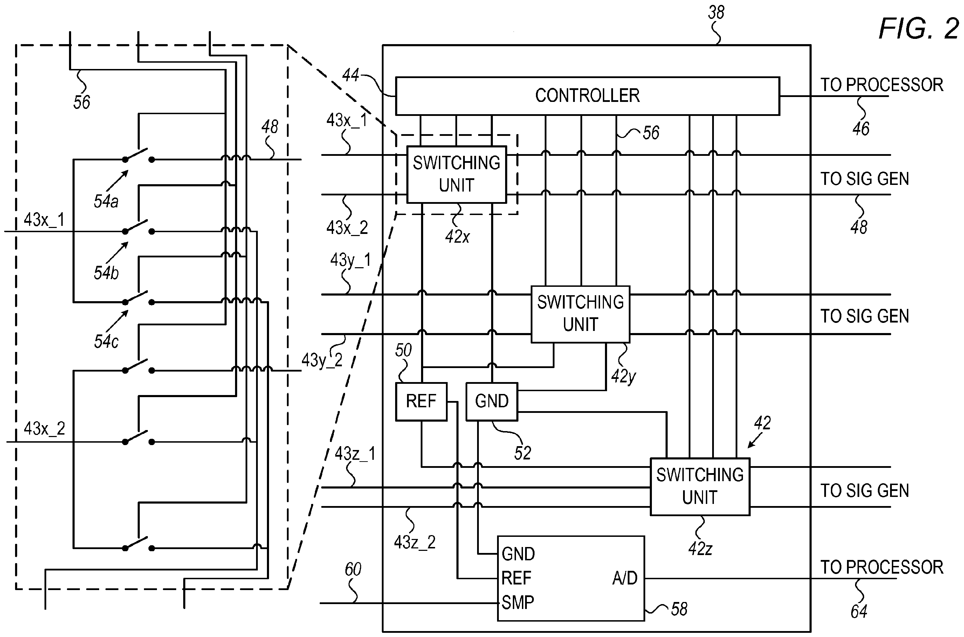

Console 32 comprises a signal generator (SIG GEN) 36, circuitry (CIRC) 38, and a processor 40. As further described below with reference to Fig. 2, circuitry 38 is configured to connect electrodes 30 in multiple different configurations, in each of which at least one pair of electrodes 30 is connected to the signal generator. For each of the configurations, processor 40 identifies the amplitude of the alternating voltage at sensing electrode 22 (referred to herein, for simplicity, as the voltage at the sensing electrode or the SEV). In particular, (i) for at least one configuration in which the x-axis pair is activated, the processor identifies an x-axis SEV, (ii) for at least one configuration in which the y-axis pair is activated, the processor identifies a y-axis SEV, and (iii) for at least one configuration in which the z-axis pair is activated, the processor identifies a z-axis SEV. The processor then ascertains the location of the sensing electrode (and hence, of the probe), based on the SEVs. In some embodiments, based on the respective locations of multiple sensing electrodes coupled to the probe, the processor further ascertains the orientation of the probe.

In addition to the aforementioned components, console 32 may comprise any other relevant components, such as an additional signal generator for generating ablating signals, or a pump for pumping irrigating fluid to the ablation site.

In general, processor 40 may be embodied as a single processor, or as a cooperatively networked or clustered set of processors. In some embodiments, the functionality of processor 40, as described herein, is implemented solely in hardware, e.g., using one or more Application-Specific Integrated Circuits (ASICs) or Field-Programmable Gate Arrays (FPGAs). In other embodiments, the functionality of processor 40 is implemented at least partly in software. For example, in some embodiments, processor 40 is embodied as a programmed digital computing device comprising at least a central processing unit (CPU) and random access memory (RAM). Program code, including software programs, and/or data are loaded into the RAM for execution and processing by the CPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

### THE CIRCUITRY

Reference is now made to Fig. 2, which is a schematic illustration of circuitry 38, in accordance with some embodiments of the present invention.

Circuitry 38 comprises an analog-to-digital (A/D) converter 58, comprising a ground (GND) terminal, a reference (REF) terminal, and a sampling (SMP) terminal. A/D converter 58 is configured to digitize (i.e., sample and quantize) the alternating voltage signal received at the sampling terminal, and/or the alternating voltage signal received at the reference terminal, with respect to the ground terminal. A digital representation of each voltage signal is output to processor 40 (Fig. 1) over an output line 64. For example, A/D converter 58 may output, to the processor, the digitized voltage signal, or simply the amplitude of the voltage signal.

Typically, circuitry 38 further comprises a respective switching unit 42 for each pair of external electrodes. In particular, the circuitry typically comprises an x-axis switching unit 42x for the x-axis pair, a y-axis switching unit 42y for the y-axis pair, and a z-axis switching unit 42z for the z-axis pair. X-axis switching unit 42x is configured to connect to the x-axis pair via respective x-axis lines 43x_1 and 43x_2, y-axis switching unit 42y is configured to connect to the y-axis pair via respective y-axis lines 43y_1 and 43y_2, and z-axis switching unit 42z is configured to connect to the z-axis pair via respective z-axis lines 43z_1 and 43z_2.

Typically, circuitry 38 further comprises a controller 44, which is configured to control switching units 42 so as to alternatingly connect the pairs of electrodes in various different configurations (or "settings"). Typically, controller 44 is connected to processor 40 over a processor-connecting line 46 such that, via line 46, the processor may cause the controller to connect the electrodes in the desired configurations. Alternatively, controller 44 may be configured to connect the electrodes in the desired configurations even without any specific input from processor 40.

Each switching unit has three settings, which differ from each other with respect to the manner in which the electrodes are connected. In particular, in an active setting, the switching unit connects the pair of electrodes to respective terminals of signal generator 36 (Fig. 1) via respective generator-connecting lines 48, such that the signal generator applies a voltage between the pair. In a reference setting, the switching unit connects the electrodes (e.g., via a reference pad 50) to the reference terminal of the A/D converter. In a ground setting, the switching unit connects the electrodes (e.g., via a ground pad 52) to the ground terminal of the A/D converter.

In some embodiments, as shown in the inset portion of Fig. 2, each switching unit comprises a respective set of switches for each of the two electrodes connected to the switching unit. The set includes a first switch 54a, which connects the electrode to the signal generator, a second switch 54b, which connects the electrode to the reference terminal, and a third switch 54c, which connects the electrode to the ground terminal. Via control lines 56, controller 44 opens and closes the switches, thus controlling the setting of the switching unit. In particular, (i) for the active setting, the controller closes first switch 54a for each of the electrodes, while keeping second switch 54b and third switch 54c open, (ii) for the reference setting, the controller closes second switch 54b for each of the electrodes, while keeping first switch 54a and third switch 54c open, and (iii) for the ground setting, the controller closes third switch 54c for each of the electrodes, while keeping first switch 54a and second switch 54b open.

In each setting, the sensing electrode is connected to the sampling terminal of A/D converter 58. For example, a sensing-electrode line 60 may connect the proximal end of wire 23 (Fig. 1) to the sampling terminal.

Typically, the frequency of the applied voltage varies between the pairs of electrodes. As a purely illustrative example, a voltage at 5.1 kHz may be applied between the x-axis pair, a voltage of 5.5 kHz between the y-axis pair, and a voltage of 5.9 kHz between the z-axis pair. The different frequencies facilitate differentiating the x-axis, y-axis, and z-axis SEVs from each other, particularly if multiple pairs of electrodes 30 are activated simultaneously.

### ASCERTAINING THE LOCATION OF THE SENSING ELECTRODE

To ascertain the location of the sensing electrode, the processor identifies the respective SEVs for multiple different settings of circuitry 38, based on the digital representation of the sampling-terminal voltage that is received, for each configuration, from the A/D converter. For example, for embodiments in which the A/D converter outputs a digital sampling-terminal voltage signal, the processor may compute the amplitude of this signal, and identify this amplitude as the SEV. For embodiments in which the A/D converter outputs the amplitude of the sampling-terminal voltage, the processor may simply identify this output as the SEV. Subsequently, based on the SEVs, the processor ascertains the location of the sensing electrode.

In some embodiments, the electrodes are connected in at least three different configurations, in each of which a different respective one of the pairs is connected to the signal generator. For example, the processor may cause the controller to execute a routine in which the pairs of electrodes are activated (i.e., connected to the signal generator such that a voltage is applied across the pair) in sequence. While each pair is activated, both of the inactive pairs are connected to the ground terminal, such that the SEV is measured with respect to the inactive pairs. Thus, a total of three SEVs are obtained: an x-axis SEV, a y-axis SEV, and a z-axis SEV. An example of such a routine is described by the following table, which shows the setting of each switching unit (SU) while each SEV is measured:

**Table 1**

| | SU 42x | SU 42y | SU 42z |
|---|---|---|---|
| x-axis SEV | ACTIVE | GROUND | GROUND |
| y-axis SEV | GROUND | ACTIVE | GROUND |
| z-axis SEV | GROUND | GROUND | ACTIVE |

Alternatively, the processor may cause the controller to execute a routine in which, in each configuration, only one of the inactive pairs is connected to the ground terminal, while the other inactive pair is connected to the reference terminal. Thus, in each configuration, the A/D converter outputs a digital representation of the voltage at the reference terminal with respect to the ground terminal. (As described above for the sampling-terminal voltage, this digital representation may include the digital reference-voltage signal that is obtained by digitizing the reference-terminal voltage, or simply the amplitude of the reference-terminal voltage.) Based on this digital representation, the processor identifies the amplitude of the voltage at the reference terminal (referred to herein, for simplicity, as the reference voltage). Subsequently to identifying the SEV and the reference voltage for each configuration, the processor ascertains the location of the sensing electrode based on the SEVs and the reference voltages, as further described below.

An example of such a routine is described by the following table:

**Table 2**

| | SU 42x | SU 42y | SU 42z |
|---|---|---|---|
| x-axis SEV | ACTIVE | REFERENCE | GROUND |
| y-axis SEV | GROUND | ACTIVE | REFERENCE |
| z-axis SEV | REFERENCE | GROUND | ACTIVE |

Another example is described by the following table:

**Table 3**

| | SU 42x | SU 42y | SU 42z |
|---|---|---|---|
| x-axis SEV | ACTIVE | GROUND | REFERENCE |
| y-axis SEV | REFERENCE | ACTIVE | GROUND |
| z-axis SEV | GROUND | REFERENCE | ACTIVE |

Alternatively, the processor may cause the controller to activate two pairs of electrodes simultaneously. An example routine that implements simultaneous activation is shown in the following table:

**Table 4**

| | SU 42x | SU 42y | SU 42z |
|---|---|---|---|
| x-axis SEV #1 | ACTIVE | ACTIVE | GROUND |
| y-axis SEV #1 | | | |
| x-axis SEV #2 | ACTIVE | GROUND | ACTIVE |
| z-axis SEV #1 | | | |
| y-axis SEV #2 | GROUND | ACTIVE | ACTIVE |
| z-axis SEV #2 | | | |

Alternatively, the processor may cause the controller to execute a routine that includes six different configurations, an example of which is shown in the following table:

**Table 5**

| | SU 42x | SU 42y | SU 42z |
|---|---|---|---|
| x-axis SEV #1 | ACTIVE | REFERENCE | GROUND |
| y-axis SEV #1 | GROUND | ACTIVE | REFERENCE |
| z-axis SEV #1 | REFERENCE | GROUND | ACTIVE |
| x-axis SEV #2 | ACTIVE | GROUND | REFERENCE |
| y-axis SEV #2 | REFERENCE | ACTIVE | GROUND |
| z-axis SEV #2 | GROUND | REFERENCE | ACTIVE |

Alternatively, any other suitable routine may be implemented.

In some embodiments, for each routine, the circuitry remains in each configuration for between 25 and 75 ms, such that, for example, a three-configuration routine lasts between 75 and 225 ms. In other embodiments, smaller or larger configuration durations may be used. Typically, during the procedure, the routine is executed repeatedly, without any break between successive executions of the routine.

### ASCERTAINING THE LOCATION

Prior to the procedure, a calibration procedure is performed, to learn the dependence of the SEV on the location of the sensing electrode.

In some embodiments, the calibration procedure utilizes another probe, which is equipped with both sensing electrode 22 and an electromagnetic sensor. The electromagnetic sensor outputs a signal, indicative of the location of the electromagnetic sensor, in the presence of a magnetic field. The other probe is moved to various different locations within the body of the subject, and at each of these locations, the x-axis, y-axis, and z-axis SEVs are ascertained, along with the location of the sensing electrode (with respect to the body of the subject) as indicated by the signal from the electromagnetic sensor. (Typically, for each SEV, the four inactive electrodes are connected to the ground terminal, as in Table 1.) Based on this information, a lookup table, which specifies the location of the sensing electrode for various triads of SEVs, is constructed.

Subsequently, during the procedure, as the SEVs are received, the processor uses the lookup table to ascertain the location of the sensing electrode. For embodiments in which reference voltages are acquired, the processor first adjusts each SEV based on the corresponding reference voltage. Typically, to perform this adjustment, the processor records the reference voltages at the beginning of the procedure, tracks any changes in the reference voltages during the procedure, and subtracts these changes from the SEVs.

For example, if the x-axis SEV is 1.1 V, but the reference voltage has increased from 0.9 V to 1 V, the processor may adjust the x-axis SEV by subtracting 0.1 V (1 V - 0.9 V) from the SEV. The adjusted x-axis SEV of 1 V may then be used (along with the adjusted y-axis and z-axis SEVs) to look up the location of the sensing electrode. For embodiments in which, for each location ascertainment, multiple SEVs are acquired for each axis, the adjusted voltages for the axis are averaged, and the averaged adjusted voltages are then used to look up the location.

## Claims

1. A system (20) for ascertaining a location of a sensing electrode (22) within a body of a subject, the system comprising:
Circuitry (38), configured to, while the sensing electrode is connected to a sampling terminal of an analog-to-digital (A/D) converter (58), connect three pairs of electrodes (30x, 30y, 30z), which are coupled to the body of the subject such that the pairs lie along different respective axes that are orthogonal to each other, in multiple different configurations, in each of which (i) at least one of the pairs is connected to respective terminals of a signal generator (36), such that the signal generator applies an alternating voltage between the pair, thus causing a sensing-electrode voltage to be attained at the sensing electrode, and (ii) at least one other one of the pairs is connected to a ground terminal of the A/D converter; and
a processor (40), configured to:
for each configuration, receive, from the A/D converter, a digital representation of a sampling-terminal voltage at the sampling terminal with respect to the ground terminal,
identify respective amplitudes of the sensing-electrode voltage for the configurations, based on the digital representation of the sampling-terminal voltage received for each configuration, and
based on the respective amplitudes of the sensing-electrode voltage, ascertain the location.

2. The system according to claim 1, wherein the configurations include three configurations, in each of which a different respective one of the pairs is connected to the signal generator.

3. The system according to claim 2, wherein, in each of the configurations, both of the pairs that are not connected to the signal generator are connected to the ground terminal.

4. The system according to claim 1,
wherein the digital representation is a first digital representation and the amplitudes are sensing-electrode-voltage amplitudes,
wherein, in each of the configurations, (i) a first one of the pairs is connected to the signal generator, (ii) a second one of the pairs is connected to the ground terminal, and (iii) a third one of the pairs is connected to a reference terminal of the A/D converter,
wherein the processor is further configured to:
for each configuration, receive, from the A/D converter, a second digital representation of a reference-terminal voltage at the reference terminal with respect to the ground terminal, and
identify respective reference-voltage amplitudes of the reference-terminal voltage for the configurations, based on the second digital representation of the reference-terminal voltage received for each configuration, and
wherein the processor is configured to ascertain the location based on the sensing-electrode-voltage amplitudes and the reference-voltage amplitudes.

5. The system according to claim 4, wherein the configurations include six configurations.

6. The system according to claim 1, wherein the circuitry comprises:
three switching units (42x, 42y, 42z), configured to connect to the pairs of electrodes, respectively; and
a controller (44), configured to connect the pairs of electrodes in the multiple different configurations by controlling the switching units.

7. The system according to claim 1, wherein the processor is further configured to cause the circuitry to connect the pairs of electrodes in the multiple different configurations.

8. A computer software product comprising a tangible non-transitory computer-readable medium in which program instructions are stored, which instructions, when read by a processor (40), cause the processor to:
while a sensing electrode (22) within a body of a subject is connected to a sampling terminal of an analog-to-digital (A/D) converter (58), cause circuitry to connect three pairs of electrodes (30x, 30y, 30z), which are coupled to the body of the subject such that the pairs lie along different respective axes that are orthogonal to each other, in multiple different configurations, in each of which (i) at least one of the pairs is connected to respective terminals of a signal generator (36), such that the signal generator applies an alternating voltage between the pair, thus causing a sensing-electrode voltage to be attained at the sensing electrode, and (ii) at least one other one of the pairs is connected to a ground terminal of the A/D converter,
for each configuration, receive, from the A/D converter, a digital representation of a sampling-terminal voltage at the sampling terminal with respect to the ground terminal,
identify respective amplitudes of the sensing-electrode voltage for the configurations, based on the digital representation of the sampling-terminal voltage received for each configuration, and
based on the respective amplitudes of the sensing-electrode voltage, ascertain a location of the sensing electrode.

9. The computer software product according to claim 8, wherein the configurations include three configurations, in each of which a different respective one of the pairs is connected to the signal generator.

10. The computer software product according to claim 9, wherein, in each of the configurations, both of the pairs that are not connected to the signal generator are connected to the ground terminal.

11. The computer software product according to claim 8,
wherein the digital representation is a first digital representation and the amplitudes are sensing-electrode-voltage amplitudes,
wherein, in each of the configurations, (i) a first one of the pairs is connected to the signal generator, (ii) a second one of the pairs is connected to the ground terminal, and (iii) a third one of the pairs is connected to a reference terminal of the A/D converter,
wherein the instructions further cause the processor to:
for each configuration, receive, from the A/D converter, a second digital representation of a reference-terminal voltage at the reference terminal with respect to the ground terminal, and
identify respective reference-voltage amplitudes of the reference-terminal voltage for the configurations, based on the second digital representation of the reference-terminal voltage received for each configuration, and
wherein the instructions cause the processor to ascertain the location based on the sensing-electrode-voltage amplitudes and the reference-voltage amplitudes.

12. The computer software product according to claim 11, wherein the configurations include six configurations.

13. The computer software product according to claim 8,
wherein the circuitry includes:
three switching units (42x, 42y, 42z), configured to connect to the pairs of electrodes, respectively, and
a controller (44), and
wherein the processor is configured to cause the controller to connect the pairs of electrodes in the multiple different configurations by controlling the switching units.

## Patentansprüche

1. System (20) zum Bestimmen eines Ortes einer Erfassungselektrode (22) in einem Körper eines Patienten, wobei das System Folgendes umfasst:
eine Schaltung (38), die, während die Erfassungselektrode mit einem Abtastanschluss eines Analog-Digital(A/D)-Wandlers (58) verbunden ist, dazu ausgelegt ist, drei Paare von Elektroden (30x, 30y, 30z), die derart an den Körper des Patienten gekoppelt sind, dass die Paare entlang verschiedenen jeweiligen Achsen liegen, die orthogonal zueinander verlaufen, in mehreren verschiedenen Auslegungen zu verbinden, wobei bei jeder (i) mindestens eines der Paare mit jeweiligen Anschlüssen eines Signalerzeugers (36) verbunden ist, derart, dass der Signalerzeuger eine Wechselspannung zwischen das Paar angelegt, wodurch bewirkt wird, dass eine Erfassungselektrodenspannung an der Erfassungselektrode erreicht wird, und (ii) mindestens ein anderes der Paare mit einem Masseanschluss des A/D-Wandlers verbunden ist; und
einen Prozessor (40), der zu Folgendem ausgelegt ist:
Empfangen einer digitalen Repräsentation einer Abtastanschlussspannung am Abtastanschluss mit Bezug auf den Masseanschluss für jede Auslegung vom A/D-Wandler,
Identifizieren von jeweiligen Amplituden der Erfassungselektrodenspannung für die Auslegungen auf Basis der digitalen Repräsentation der Abtastanschlussspannung, die für jede Auslegung erhalten wird, und
Bestimmen des Ortes auf Basis der jeweiligen Amplituden der Erfassungselektrodenspannung.

2. System nach Anspruch 1, wobei die Auslegungen drei Auslegungen beinhalten, wobei bei jeder ein anderes jeweiliges der Paare mit dem Signalerzeuger verbunden ist.

3. System nach Anspruch 2, wobei bei jeder der Auslegungen beide der Paare, die nicht mit dem Signalerzeuger verbunden sind, mit dem Masseanschluss verbunden sind.

4. System nach Anspruch 1,
wobei die digitale Repräsentation eine erste digitale Repräsentation ist und die Amplituden Erfassungselektrodenspannungsamplituden sind,
wobei bei jeder der Auslegungen (i) ein erstes der Paare mit dem Signalerzeuger verbunden ist, (ii) ein zweites der Paare mit dem Masseanschluss verbunden ist und (iii) ein drittes der Paare mit einem Referenzanschluss des A/D-Wandlers verbunden ist,
wobei der Prozessor ferner zu Folgendem ausgelegt ist:
Empfangen einer zweiten digitalen Repräsentation einer Referenzanschlussspannung am Referenzanschluss mit Bezug auf den Masseanschluss für jede Auslegung vom A/D-Wandler, und
Identifizieren von jeweiligen Referenzspannungsamplituden der Referenzanschlussspannung für die Auslegungen auf Basis der zweiten digitalen Repräsentation der Referenzanschlussspannung, die für jede Auslegung erhalten wird, und
wobei der Prozessor dazu ausgelegt ist, den Ort auf Basis der Erfassungselektrodenspannungsamplituden und der Referenzspannungsamplituden zu bestimmen.

5. System nach Anspruch 4, wobei die Auslegungen sechs Auslegungen beinhalten.

6. System nach Anspruch 1, wobei die Schaltung Folgendes umfasst:
drei Schalteinheiten (42x, 42y, 42z), die dazu ausgelegt sind, sich jeweils mit den Paaren von Elektroden zu verbinden; und
eine Steuerung (44), die dazu ausgelegt ist, die Paare von Elektroden der mehreren verschiedenen Auslegungen durch Steuern der Schalteinheiten zu verbinden.

7. System nach Anspruch 1, wobei der Prozessor ferner dazu ausgelegt ist zu bewirken, dass die Schaltung die Paare von Elektroden der mehreren verschiedenen Auslegungen verbindet.

8. Computersoftwareprodukt, das ein greifbares nichttransitorisches computerlesbares Medium umfasst, auf dem Programmanweisungen gespeichert sind, wobei die Anweisungen, wenn sie von einem Prozessor (40) gelesen werden, bewirken, dass der Prozessor Folgendes durchführt:
während eine Erfassungselektrode (22) in einem Körper eines Patienten mit einem Abtastanschluss eines Analog-Digital(A/D)-Wandlers (58) verbunden ist, Bewirken, dass eine Schaltung drei Paare von Elektroden (30x, 30y, 30z), die derart an den Körper des Patienten gekoppelt sind, dass die Paare entlang verschiedenen jeweiligen Achsen liegen, die orthogonal zueinander verlaufen, in mehreren verschiedenen Auslegungen verbindet, wobei bei jeder (i) mindestens eines der Paare mit jeweiligen Anschlüssen eines Signalerzeugers (36) verbunden ist, derart, dass der Signalerzeuger eine Wechselspannung zwischen das Paar angelegt, wodurch eine Erfassungselektrodenspannung an der Erfassungselektrode erreicht wird, und (ii) mindestens ein anderes der Paare mit einem Masseanschluss des A/D-Wandlers verbunden ist,
Empfangen einer digitalen Repräsentation einer Abtastanschlussspannung am Abtastanschluss mit Bezug auf den Masseanschluss für jede Auslegung vom A/D-Wandler,
Identifizieren von jeweiligen Amplituden der Erfassungselektrodenspannung für die Auslegungen auf Basis der digitalen Repräsentation der Abtastanschlussspannung, die für jede Auslegung erhalten wird, und
Bestimmen eines Ortes der Erfassungselektrode auf Basis der jeweiligen Amplituden der Erfassungselektrodenspannung.

9. Computersoftwareprodukt nach Anspruch 8, wobei die Auslegungen drei Auslegungen beinhalten, wobei bei jeder ein anderes jeweiliges der Paare mit dem Signalerzeuger verbunden ist.

10. Computersoftwareprodukt nach Anspruch 9, wobei bei jeder der Auslegungen beide der Paare, die nicht mit dem Signalerzeuger verbunden sind, mit dem Masseanschluss verbunden sind.

11. Computersoftwareprodukt nach Anspruch 8,
wobei die digitale Repräsentation eine erste digitale Repräsentation ist und die Amplituden Erfassungselektrodenspannungsamplituden sind,
wobei bei jeder der Auslegungen (i) ein erstes der Paare mit dem Signalerzeuger verbunden ist, (ii) ein zweites der Paare mit dem Masseanschluss verbunden ist und (iii) ein drittes der Paare mit einem Referenzanschluss des A/D-Wandlers verbunden ist,
wobei die Anweisungen ferner bewirken, dass der Prozessor Folgendes durchführt:
Empfangen einer zweiten digitalen Repräsentation einer Referenzanschlussspannung am Referenzanschluss mit Bezug auf den Masseanschluss für jede Auslegung vom A/D-Wandler, und
Identifizieren von jeweiligen Referenzspannungsamplituden der Referenzanschlussspannung für die Auslegungen auf Basis der zweiten digitalen Repräsentation der Referenzanschlussspannung, die für jede Auslegung erhalten wird, und
wobei die Anweisungen bewirken, dass der Prozessor den Ort auf Basis der Erfassungselektrodenspannungsamplituden und der Referenzspannungsamplituden bestimmt.

12. Computersoftwareprodukt nach Anspruch 11, wobei die Auslegungen sechs Auslegungen beinhalten.

13. Computersoftwareprodukt nach Anspruch 8,
wobei die Schaltung Folgendes beinhaltet:
drei Schalteinheiten (42x, 42y, 42z), die dazu ausgelegt sind, sich jeweils mit den Paaren von Elektroden zu verbinden, und
eine Steuerung (44) und
wobei der Prozessor dazu ausgelegt ist zu bewirken, dass die Steuerung die Paare von Elektroden der mehreren verschiedenen Auslegungen durch Steuern der Schalteinheiten verbindet.

## Revendications

1. Système (20) pour vérifier un emplacement d'une électrode de détection (22) dans le corps d'un sujet, le système comprenant :
des circuits (38), configurés pour, tandis que l'électrode de détection est connectée à une borne d'échantillonnage d'un convertisseur analogique-numérique (A/D) (58), connecter trois paires d'électrodes (30x, 30y, 30z), qui sont couplées au corps du sujet de telle sorte que les paires se trouvent le long de différents axes respectifs qui sont orthogonaux les uns aux autres, dans de multiples configurations différentes, dans chacune desquelles (i) au moins l'une des paires est connectée à des bornes respectives d'un générateur de signaux (36), de telle sorte que le générateur de signaux applique une tension alternative entre la paire, provoquant ainsi l'obtention d'une tension d'électrode de détection au niveau de l'électrode de détection, et (ii) au moins une autre des paires est connectée à une borne de masse du convertisseur A/D ; et
un processeur (40), configuré pour :
pour chaque configuration, recevoir, en provenance du convertisseur A/D, une représentation numérique d'une tension de borne d'échantillonnage au niveau de la borne d'échantillonnage par rapport à la borne de masse,
identifier des amplitudes respectives de la tension d'électrode de détection pour les configurations, sur la base de la représentation numérique de la tension de borne d'échantillonnage reçue pour chaque configuration, et
sur la base des amplitudes respectives de la tension d'électrode de détection, vérifier l'emplacement.

2. Système selon la revendication 1, dans lequel les configurations comprennent trois configurations, dans chacune desquelles une paire respective différente des paires est connectée au générateur de signaux.

3. Système selon la revendication 2, dans lequel, dans chacune des configurations, les deux paires qui ne sont pas connectées au générateur de signaux sont connectées à la borne de masse.

4. Système selon la revendication 1,
dans lequel la représentation numérique est une première représentation numérique et les amplitudes sont des amplitudes de tension d'électrode de détection,
dans lequel, dans chacune des configurations, (i) une première des paires est connectée au générateur de signaux, (ii) une deuxième des paires est connectée à la borne de masse, et (iii) une troisième des paires est connectée à une borne de référence du convertisseur A/D, dans lequel le processeur est en outre configuré pour :
pour chaque configuration, recevoir, en provenance du convertisseur A/D, une deuxième représentation numérique d'une tension de borne de référence au niveau de la borne de référence par rapport à la borne de masse, et
identifier des amplitudes de tension de référence respectives de la tension de borne de référence pour les configurations, sur la base de la deuxième représentation numérique de la tension de borne de référence reçue pour chaque configuration, et
dans lequel le processeur est configuré pour vérifier l'emplacement sur la base des amplitudes de tension d'électrode de détection et des amplitudes de tension de référence.

5. Système selon la revendication 4, dans lequel les configurations comprennent six configurations.

6. Système selon la revendication 1, dans lequel les circuits comprennent :
trois unités de commutation (42x, 42y, 42z), configurées pour se connecter aux paires d'électrodes, respectivement ; et
un dispositif de commande (44), configuré pour connecter les paires d'électrodes dans les multiples configurations différentes en commandant les unités de commutation.

7. Système selon la revendication 1, dans lequel le processeur est en outre configuré pour amener les circuits à connecter les paires d'électrodes dans les multiples configurations différentes.

8. Produit logiciel d'ordinateur comprenant un support lisible par ordinateur non transitoire tangible dans lequel sont stockées des instructions de programme, lesquelles instructions, lorsqu'elles sont lues par un processeur (40), amènent le processeur à :
tandis qu'une électrode de détection (22) à l'intérieur du corps d'un sujet est connectée à une borne d'échantillonnage d'un convertisseur analogique-numérique (A/D) (58), faire en sorte que les circuits connectent trois paires d'électrodes (30x, 30y, 30z), qui sont couplées au corps du sujet de telle sorte que les paires se trouvent le long de différents axes respectifs qui sont orthogonaux les uns aux autres, dans de multiples configurations différentes, dans chacune desquelles (i) au moins l'une des paires est connectée aux bornes respectives d'un générateur de signaux (36), de telle sorte que le générateur de signaux applique une tension alternative entre la paire, provoquant ainsi l'obtention d'une tension d'électrode de détection au niveau de l'électrode de détection, et (ii) au moins une autre des paires est connectée à une borne de masse du convertisseur A/D,
pour chaque configuration, recevoir, en provenance du convertisseur A/D, une représentation numérique d'une tension de borne d'échantillonnage au niveau de la borne d'échantillonnage par rapport à la borne de masse,
identifier des amplitudes respectives de la tension d'électrode de détection pour les configurations, sur la base de la représentation numérique de la tension de borne d'échantillonnage reçue pour chaque configuration, et
sur la base des amplitudes respectives de la tension de l'électrode de détection, vérifier un emplacement de l'électrode de détection.

9. Produit logiciel d'ordinateur selon la revendication 8, dans lequel les configurations comprennent trois configurations, dans chacune desquelles une paire respective différente des paires est connectée au générateur de signaux.

10. Produit logiciel d'ordinateur selon la revendication 9, dans lequel, dans chacune des configurations, les deux paires qui ne sont pas connectées au générateur de signaux sont connectées à la borne de masse.

11. Produit logiciel d'ordinateur selon la revendication 8,
dans lequel la représentation numérique est une première représentation numérique et les amplitudes sont des amplitudes de tension d'électrode de détection,
dans lequel, dans chacune des configurations, (i) une première des paires est connectée au générateur de signaux, (ii) une deuxième des paires est connectée à la borne de masse, et (iii) une troisième des paires est connectée à une borne de référence du convertisseur A/D, dans lequel les instructions amènent en outre le processeur à :
pour chaque configuration, recevoir, en provenance du convertisseur A/D, une deuxième représentation numérique d'une tension de borne de référence au niveau de la borne de référence par rapport à la borne de masse, et
identifier des amplitudes de tension de référence respectives de la tension de borne de référence pour les configurations, sur la base de la deuxième représentation numérique de la tension de borne de référence reçue pour chaque configuration, et
dans lequel les instructions amènent le processeur à vérifier l'emplacement sur la base des amplitudes de tension d'électrode de détection et des amplitudes de tension de référence.

12. Produit logiciel d'ordinateur selon la revendication 11, dans lequel les configurations comprennent six configurations.

13. Produit logiciel d'ordinateur selon la revendication 8, dans lequel les circuits comprennent :
trois unités de commutation (42x, 42y, 42z), configurées pour se connecter aux paires d'électrodes, respectivement, et
un dispositif de commande (44), et
dans lequel le processeur est configuré pour amener le dispositif de commande à connecter les paires d'électrodes dans les multiples configurations différentes en commandant les unités de commutation.
